# EUROPEAN PATENT SPECIFICATION

(11) **EP 3 946 620 B1**
(45) Date of publication and mention of the grant of the patent: **26.04.2023**
(21) Application number: 20719833.4
(22) Date of filing: 19.03.2020
(51) Int. Cl.: A61P 35/00, C07D 495/04, A61K 31/407, C07C 303/32

(54) **SALTS OF 5,6-DIHYDRO-4H-THIENO[2,3-C]PYRROL-4-ONE COMPOUND AS ERK INHIBITORS**
SALZE DER 5,6-DIHYDRO-4H-THIENO[2,3-C]PYRROL-4-ON-VERBINDUNG ALS ERK-INHIBITOREN
SELS DE COMPOSÉ 5,6-DIHYDRO-4H-THIÉNO[2,3-C]PYRROL-4-ONE UTILISÉS EN TANT QU'INHIBITEURS D'ERK

(30) Priority: 27.03.2019 US 201962824481 P
(43) Date of publication of application: 09.02.2022
(73) Proprietor: Eli Lilly and Company, Indianapolis, IN 46285 (US)
(72) Inventor: COATES, David Andrew, Indianapolis, Indiana 46206-6288 (US); JOSEPH, Sajan, Indianapolis, Indiana 46206-6288 (US); POLIZZI, Mark Andrew, Indianapolis, Indiana 46206-6288 (US); REMICK, David Michael, Indianapolis, Indiana 46206-6288 (US)
(74) Representative: Smith, Andrew George
(86) International application number: PCT/US2020/023534
(87) International publication number: WO 2020/197917

(56) References cited:
- US-A1- 2016 176 896
- RICHARD J BASTIN ET AL: "Salt Selection and Optimisation Procedures for Pharmaceutical New Chemical Entities", ORGANIC PROCESS RESEARCH AND DEVELOPMENT, AMERICAN CHEMICAL SOCIETY, US, vol. 4, no. 5, 19 July 2000 (2000-07-19), pages 427-435, XP008154792, ISSN: 1083-6160, DOI: 10.1021/OP000018U [retrieved on 2000-07-19]

## Description

The present invention provides novel crystalline salt forms of 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one useful in the treatment of certain cancers.

6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one is disclosed in US9469652 as an ERK1/2 inhibitor useful in the treatment of certain cancers. Erk has been implicated in several different types of cancer. For example, head and neck cancer, see; Rampias, T., et al., Clin Cancer Therapy, 2014, 20(11):2933-2946; for liver cancer, see Yang, S, et al., Oncology letters, 2017, 13(3):1041-1047; for breast cancer, see Bartholomeusz, C., et al., The Oncologist, 2012, 17:766-774 and Santen, R., et al., J. Steroid Biochem & Mol Bio., 2002, 80:239-256; for biliary cancer, see Marks, E., et al., World J Gastroenterology, 2016, 22(4):1335-1347; for leukemia, see Steelman, L., et al., Leukemia, 2011, 25:1080-1094; and for thyroid cancer, see Nikiforov, Y, Mod. Pathol., 2008, 21(Suppl 2):S37-S43.

The solubility of 6,6-dimethyl-2- { 2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one is much lower at elevated pH and this prevents the dissolution of the drug. Elevated stomach pH can be caused by intrinsic factors such as age, ethnicity or underlying conditions like achlorhydria. It can also be caused by extrinsic factors like vitamin/nutrient deficiencies, prior stomach surgeries, high stress levels, certain dietary supplements, certain infections, or concomitant acid reducing therapies like H2 blockers, proton pump inhibiters, or antacids. It is also common for patients undergoing oncology treatment to be prescribed proton pump therapy to manage side effects (N. R. Budha, et al., Clinical Pharmacology and Therapeutics, Aug. 2012, 92(2):203-213). As consistent exposure is required across the patient population, discovering a physically and chemically stable solid form that removes the inherent solubility limitation is desired.

The present invention provides methanesulfonic acid and 4-methylbenzenesulfonic acid salt forms of 6,6-dimethyl-2- { 2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one with improved solubility over the free base form. Certain of the compounds also have improved physical or chemical properties over the free base form, for example, solubility and tabletability.

The present invention provides a compound which is 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid. Preferably, the compound is a dihydrate.

The present invention also provides a compound which is 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid.

In an embodiment, 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid is in crystalline form. Preferably, the compound is a dihydrate. In an embodiment, 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid is in crystalline form characterized by an X-ray powder diffraction pattern (CuKα radiation, λ = 1.54060 Å) comprising a peak at 20.2, and one or more peaks at 20.9, 16.9, and 23.8 (2θ+/- 0.2°). In an embodiment, 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid dihydrate is in crystalline form characterized by an X-ray powder diffraction pattern (CuKα radiation, λ = 1.54060 Å) comprising a peak at 16.6, and one or more peaks at 15.9, 27.1, and 15.6 (2θ+/- 0.2°).

In an embodiment, 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid is in crystalline form. In an embodiment, 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid is in crystalline form characterized by an X-ray powder diffraction pattern (CuKα radiation, λ = 1.54060 Å) comprising a peak at 4.4, and one or more peaks at 22.1, 11.6, and 17.3 (2θ+/- 0.2°).

The present invention provides a pharmaceutical composition comprising 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid and one or more of a pharmaceutically acceptable carrier, diluent, or excipient. Preferably, the compound is a dihydrate. Preferably, the pharmaceutical composition (100% weight (wt) of the tablet) comprises about 65 wt% of the compound. Preferably, the pharmaceutical composition comprises about 65 wt% of the compound, about 26 wt% diluent/binder, about 5 wt% disintegrant, about 2 wt% lubricant, and about 2 wt% glidant. Preferably the pharmaceutical composition comprises about 65 wt% of the compound, about 26 wt% microcrystalline cellulose, about 5 wt% croscarmellose sodium, about 2 wt% sodium stearyl fumarate, and about 2 wt% silicone dioxide.

The present invention provides a pharmaceutical composition comprising is 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid and one or more of a pharmaceutically acceptable carrier, diluent, or excipient.

The present invention provides 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid for use in therapy. The present invention also provides 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid for use in the treatment of cancer. The present invention also provides 6,6-dimethyl-2-{2-[(1-methyl-1H-pyrazol-5-yl)amino]pyrimidin-4-yl }-5-[2-(morpholin-4-yl)ethyl]-5, 6-dihydro-4H-thieno[2,3-c]pyrrol-4-one; methanesulfonic acid for use in the treatment of melanoma, colorectal cancer, pancreatic cancer, non-small cell lung cancer, head and neck cancer, liver cancer, breast cancer, biliary cancer, leukemia, or thyroid cancer. Preferably, the compound is a dihydrate. The present invention provides 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid for use in therapy. The present invention also provides 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid for use in the treatment of cancer. The present invention also provides 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid for use in the treatment of melanoma, colorectal cancer, pancreatic cancer, non-small cell lung cancer, head and neck cancer, liver cancer, breast cancer, biliary cancer, leukemia, or thyroid cancer.

As used herein, the term "patient" refers to a human.

As used herein, the term "treating" (or "treat" or "treatment") refers to the process involving a slowing, interrupting, arresting, controlling, reducing, or reversing the progression or severity of a symptom, disorder, condition, or disease such as cancer.

A compound of the present invention may be prepared by a variety of procedures known in the art, some of which are illustrated in the Preparations and Examples below.

The specific synthetic steps for each of the routes described may be combined in different ways, or in conjunction with steps from different procedures, to prepare the compound as described herein. The products can be recovered by conventional methods well known in the art, including extraction, evaporation, precipitation, chromatography, filtration, trituration, and crystallization. The reagents and starting materials are readily available to one of ordinary skill in the art.

Unless specifically noted, abbreviations used herein are defined according to Aldrichimica Acta, Vol. 17, No. 1, 1984. Other abbreviations are defined as follows: "ACN" refers to acetonitrile; "API" refers to active pharmaceutical ingredients; "DCM" refers to dichloromethane or methylene chloride; "EtOAc" refers to ethyl acetate; "EtOH" refers to ethanol or ethyl alcohol; "FaSSIF" refers to fasted state simulated intestinal fluid; "FedSSIF" refers to Fed state simulated intestinal fluid; "hr" or "hrs" refers to hour or hours; "IPA" refers to isopropanol or isopropyl alcohol; "kN" refers to kilo newtons; "MeOH" refers to methanol or methyl alcohol; "mesylate" refers to methanesulfonic acid; "MPa" refers to Mega Pascal; "PK" refers to pharmacokinetics; "pKa" refers to acid dissociation constant; "PPI" refers to proton pump inhibitor; "RH" refers to relative humidity; "rpm" refers to revolution per minute; "SGF" refers to simulated gastric fluid; "TFA" refers to trifluoroacetic acid; "THF" refers to tetrahydrofuran and "tosylate" refers to 4-methylbenzenesulfonic acid or *p*-toluenesulfonic acid.

### Preparations and Examples

The following Preparations and Examples further illustrate the invention.

6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one may be prepared as described in US9469652 and can also be identified as the free base herein.

The following Preparations can also be used as intermediates in the preparation of 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one and salt Examples of 6,6-dimethyl-2-{2-[(1-methyl-1H-pyrazol-5-yl)amino]pyrimidin-4-yl }-5-[2-(morpholin-4-yl)ethyl]-5,6-dihydro-4H-thieno[2,3-c]pyrrol-4-one which are described below.

### Preparation 1

### 6,6-Dimethyl-5-(2-morpholinoethyl)-5,6-dihydro-4H-thieno[2,3-c]pyrrol-4-one

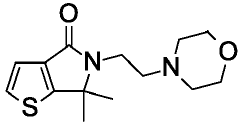

2-(2-Hydroxypropan-2-yl)thiophene-3-carboxylic acid (6.5 g, 34.9 mmol) is dissolved in MeOH (49.5 mL, Note: Water can also be used as the reaction solvent with similar results.), followed by the addition of 2-morpholinoethan-1-amine (5.5 mL, 41.9 mmol). The resulting mixture is sealed in a reactor and heated to 140 °C for 21.0 hrs. The reactor is cooled and the reaction mixture is concentrated to give the title compound as an oil (10.8 g, 89%, about 81% potency). The crude title compound is dissolved in IPA (104 mL) with warming and heated with stirring to 50 °C. L-Tartaric acid (4.7 g, 31.2 mmol) is dissolved in IPA (100 mL) and added to the mixture over 0.5 hr. The resulting slurry is heated to 75 °C briefly and then cooled to 22 °C over 2.0 hrs. The solids are filtered and washed with IPA (100 mL) and dried in a vacuum oven at 45 °C to give the title compound as an L-tartrate salt as a white crystalline solid (11.6 g, 86%). 6,6-Dimethyl-5-(2-morpholinoethyl)-5,6-dihydro-4H-thieno[2,3-c]pyrrol-4-one L-tartrate is dissolved in a mixture of DCM and 1 M NaOH and the organic phase separated and washed with aqueous sodium bicarbonate and brine. The organic phase is dried with Na₂SO₄ and concentrated to give the title compound in essentially quantitative yield from the tartrate salt as a thick oil that crystallizes on standing to produce a waxy solid. MS (m/z: 281.1 (M+H)).

### Preparation 2

### 2-Bromo-6,6-dimethyl-5-(2-morpholinoethyl)-5,6-dihydro-4H-thieno[2,3-c]pyrrol-4-one hydrobromide

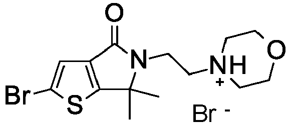

6,6-Dimethyl-5-(2-morpholinoethyl)-5,6-dihydro-4H-thieno[2,3-c]pyrrol-4-one (0.5 g, 1.78 mmol) is dissolved in MeOH (5 mL). The mixture is warmed to 45 °C and bromine (0.37 mL, 7.12 mmol) is added in portions over about 2.0 hrs. The resulting slurry is cooled to 22 °C and the solids filtered, washed with IPA and dried to give the title compound (0.46 g, 58%). MS (m/z: 359.0 (M+H)).

### Preparation 3

### Dimethyl {6,6-dimethyl-5-[2-(morpholin-4-yl)ethyl]-4-oxo-5,6-dihydro-4H-thieno[2,3-c]pyrrol-2-yl}boronate

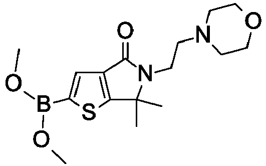

2-Bromo-6,6-dimethyl-5-(2-morpholinoethyl)-5,6-dihydro-4H-thieno[2,3-c]pyrrol-4-one (200 g, 556.67 mmol) is combined with THF (3 L) under a nitrogen atmosphere at 15 to 30 °C and the resulting mixture stirred for 0.5 hr. The mixture is cooled to 0 to 10 °C and trimethyl borate (86.77 g, 835.05 mmol) is added followed by 2 M isopropylmagnesium chloride in THF (419.42 mL, 835.05 mmol). The intermediate is used directly without isolation.

### Example 1

### 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid

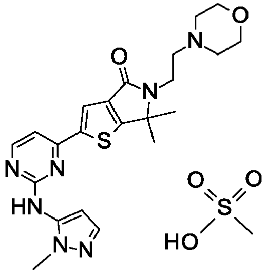

6,6-Dimethyl-2-{2-[(1 -methyl-1H-pyrazol-5-yl)amino]pyrimidin-4-yl }-5-[2-(morpholin-4-yl)ethyl]-5,6-dihydro-4H-thieno[2,3-c]pyrrol-4-one (10.0 g, 22 mmol) is suspended in acetone (100 mL) and stirred at 60 °C at 1000 rpm. Methanesulfonic acid (2.4 g, 1.600 mL, 25 mmol) is added slowly. The mixture is stirred for 60 minutes at 60 °C and results in a thick off white slurry. The solid is collected by filtration and dried at 80 °C for 2 hrs to give the title compound (12.1 g, 98.9%).

### X-Ray Powder Diffraction (XRD)

The XRPD patterns of crystalline solids are obtained on a Bruker D4 Endeavor X-ray powder diffractometer, equipped with a CuKα source and a Vantec detector, operating at 35 kV and 50 mA. The sample is scanned between 4 and 40 2θ°, with a step size of 0.008 2θ° and a scan rate of 0.5 seconds/step, and using 1.0 mm divergence, 6.6 mm fixed anti-scatter, and 11.3 mm detector slits. The dry powder is packed on a quartz sample holder and a smooth surface is obtained using a glass slide. The crystal form diffraction patterns are collected at ambient temperature and relative humidity. Crystal peak positions are determined in MDI-Jade after whole pattern shifting based on an internal NIST 675 standard with peaks at 8.853 and 26.774 2θ°. It is well known in the crystallography art that, for any given crystal form, the relative intensities of the diffraction peaks may vary due to preferred orientation resulting from factors such as crystal morphology and habit. Where the effects of preferred orientation are present, peak intensities are altered, but the characteristic peak positions of the polymorph are unchanged. See, *e.g.* The United States Pharmacopeia #23, National Formulary #18, pages 1843-1844, 1995. Furthermore, it is also well known in the crystallography art that for any given crystal form the angular peak positions may vary slightly. For example, peak positions can shift due to a variation in the temperature at which a sample is analyzed, sample displacement, or the presence or absence of an internal standard. In the present case, a peak position variability of ± 0.2 2θ° is presumed to take into account these potential variations without hindering the unequivocal identification of the indicated crystal form. Confirmation of a crystal form may be made based on any unique combination of distinguishing peaks.

A prepared sample of crystalline 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid is characterized by an XRD pattern using CuKα radiation as having diffraction peaks (2-theta values) as described in Table 1 below, and in particular having peaks at 20.2 in combination with one or more of the peaks selected from the group consisting of 20.9, 16.9, and 23.8; with a tolerance for the diffraction angles of 0.2 degrees.

**Table 1: X-ray Powder Diffraction Peaks of the Crystalline Example 1**

| | Example 1 Peak Positions | |
|---|---|---|
| Peak | Angle (°2-Theta) +/- 0.2° | Relative Intensity (% of most intense peak) |
| 1 | 12.3 | 25.3% |
| 2 | 16.0 | 24.3% |
| 3 | 16.9 | 49.8% |
| 4 | 19.2 | 25.1% |
| 5 | 20.2 | 100.0% |
| 6 | 20.9 | 52.6% |
| 7 | 21.9 | 28.9% |
| 8 | 23.8 | 31.6% |
| 9 | 25.5 | 15.0% |
| 10 | 27.1 | 21.4% |

### Alternate Preparation of Example 1

### 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid

Methanesulfonic acid (110 mg, 1.14 mmol) is added to a solution of 6,6-dimethyl-2-{2-[(1-methyl-1H-pyrazol-5-yl)amino]pyrimidin-4-yl }-5-[2-(morpholin-4-yl)ethyl]-5,6-dihydro-4H-thieno[2,3-c]pyrrol-4-one (518 mg, 1.14 mmol) in a mixture of MeOH (6 mL) and DCM (6 mL). The mixture is sonicated for 30 minutes at room temperature. The reaction is concentrated under vacuum to give the title compound (633 mg, 100%). MS (m/z): 454 (M+1).

### Example 2

### 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid dihydrate

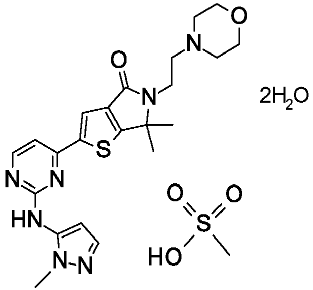

6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one (16.07 g, 35.4 mmol) is suspended in 90% ethanol, stirred and heated to 70 °C. Additional ethanol (100 mL) is added to give a solution. Methanesulfonic acid (3.75 g, 39 mmol) is added dropwise and rinsed in with 90% ethanol (2 mL). The mixture is cooled to 60 °C and seeds of the title compound are added which dissolved upon addition. The addition of seeds is repeated at 50 °C. The mixture is stirred at 50 °C for 2 hrs and then cooled to room temperature. The resulting precipitate is isolated by filtration and allowed to air dry to give the title compound (13.4 g, 70%).

A prepared sample of crystalline 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid dihydrate is characterized by an XRD pattern using CuKα radiation as having diffraction peaks (2-theta values) as described in the Table 2 below, and in particular having peaks at 16.6 in combination with one or more of the peaks selected from the group consisting of 15.9, 27.1, and 15.6; with a tolerance for the diffraction angles of 0.2 degrees.

**Table 2: X-ray Powder Diffraction Peaks of Crystalline Example**

| | Example 2 Peak Positions | |
|---|---|---|
| Peak | Angle (°2-Theta) +/- 0.2° | Relative Intensity (% of most intense peak) |
| 1 | 10.2 | 26.0% |
| 2 | 12.1 | 29.9% |
| 3 | 15.6 | 91.2% |
| 4 | 15.9 | 96.8% |
| 5 | 16.6 | 100.0% |
| 6 | 18.5 | 40.6% |
| 7 | 21.0 | 81.9% |
| 8 | 23.5 | 71.2% |
| 9 | 24.5 | 32.2% |
| 10 | 27.1 | 94.8% |

### Example 3

### 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid

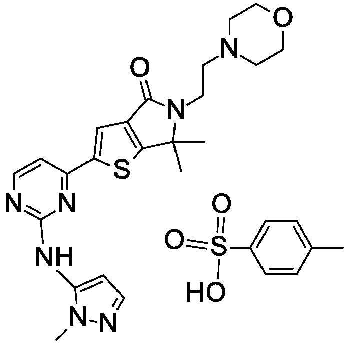

6,6-Dimethyl-2-{2-[(1 -methyl-1H-pyrazol-5-yl)amino]pyrimidin-4-yl }-5-[2-(morpholin-4-yl)ethyl]-5,6-dihydro-4H-thieno[2,3-c]pyrrol-4-one (1125 mg, 2.5 mmol) in 95% ethanol (10 mL) and the mixture is stirred at 70 °C at 1000 rpm. 4-Toluenesulfonic acid (460 mg, 2.7 mmol) is dissolved in EtOAc (5 mL) and the initial slurry becomes a gummy yellow solid. The mixture is stirred for 30 minutes and a white solid results. The solid is collected by filtration and dried at 60 °C under vacuum to give the title compound.

A prepared sample of crystalline 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid is characterized by an XRD pattern using CuKα radiation as having diffraction peaks (2-theta values) as described in Table 3 below, and in particular having peaks at 4.4 in combination with one or more of the peaks selected from the group consisting of 22.1, 11.6, and 17.3; with a tolerance for the diffraction angles of 0.2 degrees.

**Table 3: X-ray Powder Diffraction Peaks of Crystalline Example 3**

| | Example 3 Peak Positions | |
|---|---|---|
| Peak | Angle (°2-Theta) +/- 0.2° | Relative Intensity (% of most intense peak) |
| 1 | 4.4 | 100.0% |
| 2 | 11.6 | 23.9% |
| 3 | 12.8 | 16.1% |
| 4 | 13.6 | 16.0% |
| 5 | 14.3 | 21.3% |
| 6 | 17.3 | 23.1% |
| 7 | 18.2 | 19.4% |
| 8 | 22.1 | 40.3% |
| 9 | 26.1 | 18.4% |
| 10 | 27.4 | 21.6% |

### Alternate Preparation of Example 3

### 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid

4-Methylbenzenesulfonic acid monohydrate (216 mg, 1.14 mmol) is dissolved in DCM (3 mL) and added to a solution of 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one (516 mg, 1.14 mmol) in DCM (3 mL). Sonicate the mixture for 30 minutes at room temperature. Concentrate the reaction under vacuum to give the title compound (708 mg, 100%). MS (m/z): 454 (M+1) and 171 (M+1).

### Reference Example 4

### 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; hydrochloride

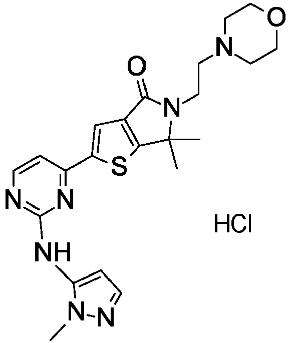

Concentrated hydrochloric acid (10 mL, 120 mmol) is slowly added to a solution of 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one (500 mg, 1.10 mmol) in MeOH (50 mL). The mixture is sonicated for 20 minutes at room temperature. The reaction is concentrated under vacuum to give the title compound (531 mg, 98%). MS (m/z): 454 (M+1).

### Assays

### Solubility

Salts (crystalline) are expected to have lower solubility at low pH, but may maintain higher solubility if they are metastable with respect to the free base solubility near or above the pKa. The extent of stability is not predictable, but must be ascertained from dissolution and solubility experiments.

The purpose of this assay is to measure the solubility of certain solid forms of the present invention in comparison to 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one (free base). pKa Determination is performed using a UV-metric method. The sample is titrated in a fast-UV triple titration between pH 2-12 at a concentration of 31-19 µM under aqueous condition. Subsequently, the sample is titrated in three UV-metric titrations from pH 1.5-10 at a concentration of 30-27 µM with no precipitation and the observed pKa averages are reported.

To determine the solubility of the three different crystal forms, a single aliquot of the test media (2 mL, see table 4) is added to an accurately weighed sample. Samples are mixed at room temperature for a period of 24 hours. If a solution is observed, more crystalline material is added and the suspension is slurried for at least 2 hours, after which, an aliquot is withdrawn, filtered through a 0.45 µm PTFE filter and characterized by HPLC using the following HPLC conditions: Column Waters XBridge Phenyl, 4.6 x 100 mm, 3.5 µm, mobile phase A is 0.% NH₄OH in water, mobile phase B is MeOH, diluent is 75% ACN and 25% water, gradient is 50% A at time 0, 25% A at time 10-13 minutes, and 50% A at time 13.1 to 18 minutes with a flow rate of 0.9 mL/minute, a column temperature of 35 °C, UV at 225 nM, an injection volume of 5 µL, and autosampler at ambient temperature. The remaining solids are air dried prior to analysis by XRPD. One replicate from each sample has triplicate HPLC analysis. pH Measurement of the solution is performed using calibrated scientific pH equipment. 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one (free base) has two basic pKa sites of 2.3 and 6.3 within the relevant physiological pH range. The equilibrium solubility in simulated intestinal fluids of the free base (assumed crystalline) compared to the crystalline methanesulfonic acid dihydrate salt (Example 2) and 4-methylbenzenesulfonic acid salt (Example 3), (assumed crystalline) are shown in Table 4.

**Table 4: Equilibrium Solubilities for Crystalline Solid Forms in Simulated Intestinal Fluids**

| Media | Free Base | | Methanesulfonic acid dihydrate, Example 2 | | 4-Methylbenzenesulfonic acid, Example 3 | |
|---|---|---|---|---|---|---|
| | mg/mL | pH | mg/mL | pH | mg/mL | pH |
| SGF | 3.70 | 5.05 | >9.70 | 2.62 | 7.08 | 2.46 |
| FaSSIF | 0.13 | 6.70 | >19.00 | 5.33 | 12.24 | 5.44 |
| FedSSIF | 5.65 | 5.36 | >20.34 | 5.09 | 13.49 | 5.04 |

These results indicate that the free base has a prototypical free base solubility behavior with higher solubility at low pH and lower solubility at higher pH *See, e.g.,* P. Stahl, et al., Handbook of Pharmaceutical Salts: Properties, Selection and Use, VCHA/Wiley-VCH, revised 2nd ed. Furthermore, both the methanesulfonic acid dihydrate (Example 2) and 4-methylbenzenesulfonic acid (Example 3) salt forms have higher solubility in FaSSIF and FedSSIF compared to the free base. Additionally, the methanesulfonic acid dihydrate salt (Example 2) is physically stable with respect to solution mediated form conversion in FaSSIF and FedSSIF.

### Modeling

Based on clinical modeling studies (E. Kosewicz, et al., European J Pharma Sciences, 16 June 2014, 57:300-321), the superior *in-vitro* solubility of the methanesulfonic acid dihydrate salt (Example 2) will improve the exposure for patients on any PPI or those who may have an otherwise elevated stomach pH. Example 2 removes the expected human dose dependent fraction absorbed (Fa) of the free base up to at least a 600 mg oral dose.

### Physical Stability

Physical stability is an important attribute not only with respect to ensuring dissolution and solubility, but also for API and dosage form drug development and manufacturing operations (drying, storage, shipping transfers, etc.). Not all crystalline salts have the needed physical properties to enable drug development.

The purpose of this assay is to determine the hygroscopicity of the solid forms of the present invention under conditions normally encountered in manufacturing and storage of drug products. Moisture analysis is performed at 25 °C using a TA instrument. The following experimental conditions are used: sample size -3-20 mg, dry sample at 0% RH until <0.0 weight change over 10 minutes, 5-95% RH adsorption/desorption range, and 5% RH step interval. The minimum equilibration criterion is <0.01% weight change for 5 minutes. Table 5 describes the hygroscopicity profile of Examples 1 through 4.

**Table 5: Hygroscopicity studies on Example 1-4.**

| Compound | 20% RH | 60% RH | 90% RH |
|---|---|---|---|
| Example 1 | <0.1 wt% increase | <0.1 wt% increase | ∼4 wt. % increase with conversion to another form at 95% RH |
| Example 2 | <0.1 wt% increase | <0.1 wt% increase | <0.1 wt% increase |
| Example 3 | <0.1 wt% increase | ∼0.2 wt% increase | ∼0.5 wt. % increase |
| Example 4 | ∼0.4 wt. % increase | ∼6 wt% increase | Conversion to another form |

The results of these studies indicate that the methanesulfonic acid dihydrate salt (Example 2) and the 4-methylbenzenesulfonic anhydrous salt (Example 3) have preferred hygroscopicity profiles for drug product manufacturing processes.

### Compaction Properties

The compaction properties of the API aids in effective production of the tablet with high drug loading (C. C. Sun, et al., J Pharm Sciences, Jan 2009, 98(1):239-247.

The purpose of this study is to determine the compaction properties of certain solid forms of the present invention. The experimental compaction testing is evaluated on Instron Universal Testing System equipped with a 50 kN load cell using Natoli 10 mm flat round punches and die for material compression. The profiles are generated at 5, 10, and 15 kN with a target tablet weight of 300 mg for each compact. The three compacts are made at each pressure set point while the Instron is traveling at 50 mm/minute. The compacts final weight is measured on a balance. The tablet's diameter and thickness is measured using Mitutoyo Thickness Gauge and hardness testing is completed on a Vankel Hardness tester using axially breakage. Table 6 shows the tensile strength of methanesulfonic acid dihydrate salt (Example 2) and 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one (free base).

**Table 6: Tabletability Properties**

| Sample | Pressure (MPa) | Tensile (MPa) |
|---|---|---|
| Example 2 | 64 | 1.2034 |
| Example 2 | 127 | 2.1532 |
| Example 2 | 191 | 2.4082 |
| Free base | 64 | 0.5415 |
| Free base | 127 | 1.0491 |
| Free base | 191 | 1.6394 |
| Mannitol SD200 | 64 | 0.4873 |
| Mannitol SD200 | 127 | 1.1966 |
| Mannitol SD200 | 191 | 2.1411 |
| Lactose Anhydrous | 64 | 0.9687 |
| Lactose Anhydrous | 128 | 1.9119 |
| Lactose Anhydrous | 191 | 3.1671 |

The results of this study indicate that the compaction properties of methanesulfonic acid dihydrate salt (Example 2) are in range with excipients such as mannitol SD200 and Lactose Anhydrous commonly used to make strong tablets in manufacturing.

### Formulation Assessment

### Formulation

The physical properties of API powder flowability, compressibility, and reduced sticking to tableting tools enables the manufacture of direct-compression prototype formulations at very high API concentrations.

The purpose of this study is to evaluate the properties of certain solid forms of the present invention with respect to the suitability for manufacturing high drug load tablets. The API flowability is measured using a United States Pharmacopeia method for bulk/tapped density and a powder rheometer. The formula contains ∼64.6 wt% Example 2, 26.4 wt% microcrystalline cellulose (diluent/binder), 5 wt% croscarmellose sodium (disintegrant), 2 wt% sodium stearyl fumarate (lubricant), and 2 wt% silicone dioxide (glidant). The components are combined and tumble blended in a single step at a 40 g scale. The resulting blend is then tested using the same bulk/tapped density and powder rheometry tests. The blend is compressed on a single-station, instrumented tablet press. The accelerated studies are comprised of prototype tablets of methanesulfonic acid dihydrate (Example 2) at a 50% free base equivalence drug load. The tablets are prepared and stored in open-dish containers to evaluate stability. Samples are stored at 40 °C/11% RH, 40 °C/75% RH, 50 °C/22% RH, and 50 °C/50% RH. At designated time points the samples are characterized by XRPD for physical form and by HPLC using the following conditions: Column Zorbax Bonus-RP, 3.5-micron, 4.6 x 75 mm, mobile phase A is 0.1% TFA in water, mobile phase B is 0.1% TFA in ACN, gradient is 95% A at time 0 to 23% A at 9.5 minutes-12.1 minutes, 5% A at time 13-16 minutes, and 95% A at time 16.1 to 20 minutes with a flow rate of 1.5 mL/minute, a column temperature of 30 °C, UV at 227 nM, an injection volume of 5 µL, and autosampler at ambient temperature. The drug product formulation stress study under accelerated open dish conditions for methanesulfonic acid dihydrate (Example 2) is depicted in Table 5.

**Table 5: Physical Stability of Example 2 in Prototype Tablet**

| Time | 40 °C/11% RH | 40 °C/75% RH | 50 °C/22% RH | 50 °C/50% RH |
|---|---|---|---|---|
| Plastic Bottles | Open Dish | Open Dish | Open Dish | Open Dish |
| 7 days | Stable | Stable | Stable | Stable |
| 14 days | Stable | Stable | Stable | Stable |
| 30 days | -- | Stable | Stable | Stable |

The results of this study indicate, surprisingly, that the physical properties of methanesulfonic acid dihydrate (Example 2) compared to the free base yielded improved powder flowability, compressibility, and reduced sticking to tableting tools. Combined, these properties enable a manufacturable, direct-compression prototype formulation at very high API concentration (65 wt% Example 2, 50 wt% free base equivalent). Typically, this API concentration requires a granulation unit operation prior to compression.

## Claims

1. A compound which is 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid.

2. The compound according to claim 1 wherein the compound is crystalline.

3. The compound according to claim 2 which is **characterized by** an X-ray powder diffraction pattern (CuKα radiation, λ = 1.54060 Å) comprising a peak at 20.2, and one or more peaks at 20.9, 16.9, and 23.8 (2θ+/- 0.2°).

4. A compound which is 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; methanesulfonic acid dihydrate.

5. The compound according to claim 4 wherein the compound is crystalline.

6. The compound according to claim 5 which is **characterized by** an X-ray powder diffraction pattern (CuKα radiation, λ = 1.54060 Å) comprising a peak at 16.6, and one or more peaks at 15.9, 27.1, and 15.6 (2θ+/- 0.2°).

7. A compound which is 6,6-dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-one; 4-methylbenzenesulfonic acid.

8. The compound according to claim 7 wherein the compound is crystalline.

9. The compound according to claim 8 which is **characterized by** an X-ray powder diffraction pattern (CuKα radiation, λ = 1.54060 Å) comprising a peak at 4.4, and one or more peaks at 22.1, 11.6, and 17.3 (2θ+/- 0.2°).

10. A pharmaceutical composition comprising a compound according to any one of claims 1 to 9 and one or more of a pharmaceutically acceptable carrier, diluent, or excipient.

11. The pharmaceutical composition according to claim 10 wherein the composition comprises about 65 wt% of the compound.

12. A compound according to any one of claims 1 to 9 for use in therapy.

13. The compound according to any one of claims 1 to 9 for use in the treatment of cancer.

14. The compound for use according to claim 13 wherein the cancer is melanoma, colorectal cancer, pancreatic cancer, non-small cell lung cancer, head and neck cancer, liver cancer, breast cancer, biliary cancer, leukemia, or thyroid cancer.

## Patentansprüche

1. Verbindung, bei der es sich um 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-on; Methansulfonsäure handelt.

2. Verbindung nach Anspruch 1, wobei die Verbindung kristallin ist.

3. Verbindung nach Anspruch 2, **gekennzeichnet durch** ein Röntgenpulver-Beugungsmuster (CuKa-Strahlung, λ = 1,54060 Å), welches einen Peak bei 20,2 und einen oder mehrere Peaks bei 20,9, 16,9 und 23,8 (2θ+/- 0,2°) aufweist.

4. Verbindung, bei der es sich um 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-on; Methansulfonsäuredihydrat handelt.

5. Verbindung nach Anspruch 4, wobei die Verbindung kristallin ist.

6. Verbindung nach Anspruch 5, **gekennzeichnet durch** ein Röntgenpulver-Beugungsmuster (CuKa-Strahlung, λ = 1,54060 Å), welches einen Peak bei 16,6 und einen oder mehrere Peaks bei 15,9, 27,1 und 15,6 (2θ+/- 0,2°) aufweist.

7. Verbindung, bei der es sich um 6,6-Dimethyl-2-{2-[(1-methyl-1H-pyrazol-5-yl)amino]pyrimidin-4-yl}-5-[2-(morpholin-4-yl)ethyl]-5,6-dihydro-4H-thieno[2,3-c]pyrrol-4-on; 4-Methylbenzolsulfonsäure handelt.

8. Verbindung nach Anspruch 7, wobei die Verbindung kristallin ist.

9. Verbindung nach Anspruch 8, **gekennzeichnet durch** ein Röntgenpulver-Beugungsmuster (CuKa-Strahlung, λ = 1,54060 Å), das einen Peak bei 4,4 und einen oder mehrere Peaks bei 22,1, 11,6 und 17,3 (2θ+/-0,2°) aufweist.

10. Pharmazeutische Zusammensetzung, aufweisend eine Verbindung nach einem der Ansprüche 1 bis 9 und einen oder mehrere eines pharmazeutisch annehmbaren Trägers, Verdünnungsmittels oder Arzneiträgerstoffes.

11. Pharmazeutische Zusammensetzung nach Anspruch 10, wobei die Zusammensetzung etwa 65 Gew.-% der Verbindung umfasst.

12. Verbindung nach einem der Ansprüche 1 bis 9 zur Verwendung in der Therapie.

13. Verbindung nach einem der Ansprüche 1 bis 9 zur Verwendung bei der Behandlung von Krebs.

14. Verbindung zur Verwendung nach Anspruch 13, wobei es sich bei dem Krebs um ein Melanom, kolorektales Karzinom, Pankreaskarzinom, nichtkleinzelligen Lungenkarzinom, Kopf-Halskarzinom, Leberkarzinom, Mammakarzinom, Gallenkrebs, Leukämie oder Schilddrüsenkarzinom handelt.

## Revendications

1. Composé qui est la 6,6-diméthyl-2-{2-[(1-méthyl-1H-pyrazol-5-yl)amino]pyrimidin-4-yl}-5-[2-(morpholin-4-yl)éthyl]-5,6-dihydro-4H-thiéno[2,3-c]pyrrol-4-one ; acide méthanesulfonique.

2. Composé selon la revendication 1, dans lequel le composé est cristallin.

3. Composé selon la revendication 2, qui est **caractérisé par** un diagramme de diffraction des rayons X sur poudre (rayonnement CuKa, λ = 1,54060 Å) comprenant un pic à 20,2, et un ou plusieurs pics à 20,9, 16,9 et 23,8 (2θ +/- 0,2 °).

4. Composé qui est la 6,6-diméthyl-2-{2-[(1-méthyl-1H-pyrazol-5-yl)amino]pyrimidin-4-yl}-5-[2-(morpholin-4-yl)éthyl]-5,6-dihydro-4H-thiéno[2,3-c]pyrrol-4-one ; acide méthanesulfonique dihydraté.

5. Composé selon la revendication 4, dans lequel le composé est cristallin.

6. Composé selon la revendication 5, qui est **caractérisé par** un diagramme de diffraction des rayons X sur poudre (rayonnement CuKa, λ = 1,54060 Å) comprenant un pic à 16,6, et un ou plusieurs pics à 15,9, 27,1 et 15,6 (20 +/- 0,2 °).

7. Composé qui est la 6,6-diméthyl-2-{2-[(1-méthyl-1H-pyrazol-5-yl)amino]pyrimidin-4-yl}-5-[2-(morpholin-4-yl)éthyl]-5,6-dihydro-4H-thiéno[2,3-c]pyrrol-4-one ; acide 4-méthylbenzènesulfonique.

8. Composé selon la revendication 7, dans lequel le composé est cristallin.

9. Composé selon la revendication 8 qui est **caractérisé par** un diagramme de diffraction des rayons X sur poudre (rayonnement CuKa, λ = 1,54060 Å) comprenant un pic à 4,4, et un ou plusieurs pics à 22,1, 11,6 et 17,3 (20 +/- 0,2 °).

10. Composition pharmaceutique comprenant un composé selon l'une quelconque des revendications 1 à 9 et un ou plusieurs éléments parmi un support, diluant ou excipient pharmaceutiquement acceptable.

11. Composition pharmaceutique selon la revendication 10, dans laquelle la composition comprend environ 65 % en poids du composé.

12. Composé selon l'une quelconque des revendications 1 à 9 pour une utilisation en thérapie.

13. Composé selon l'une quelconque des revendications 1 à 9 pour une utilisation dans le traitement du cancer.

14. Composé pour une utilisation selon la revendication 13, dans lequel le cancer est le mélanome, le cancer colorectal, le cancer du pancréas, le cancer du poumon non à petites cellules, le cancer de la tête et du cou, le cancer du foie, le cancer du sein, le cancer des voies biliaires, la leucémie ou le cancer de la thyroïde.
